# EUROPÄISCHE PATENTSCHRIFT

(11) **EP 0 455 188 B1**
(45) Veröffentlichungstag und Bekanntmachung des Hinweises auf die Patenterteilung: **11.10.1995**
(21) Anmeldenummer: 91106904.5
(22) Anmeldetag: 27.04.1991
(51) Int. Cl.: A61B 17/39

(54) **Koagulationsinstrument zum Stillen von Blutungen in Nasenhöhlen**
Apparatus for coagulation of bleedings in the nasal cavities
Appareil de coagulation d'hémmoragies dans les cavités nasales

(30) Priorität: 04.05.1990 DE 4014350
(43) Veröffentlichungstag der Anmeldung: 06.11.1991
(73) Patentinhaber: Richard Wolf GmbH, 75438 Knittlingen (DE)
(72) Erfinder: Wurman, Leonard, Troy Wausau, Wisconsin 55401 (US); Heckele, Helmut, W-7134 Knittlingen (DE)
(74) Vertreter: Wilcken, Thomas, Dipl.-Ing.

(56) Entgegenhaltungen:
- DE-A- 2 426 781
- US-A- 2 382 109
- US-A- 2 395 631
- US-A- 3 939 840
- US-A- 4 532 924
- US-A- 4 682 596
- Katalogblätter G 20, V. 88 und G 21, V. 88 der Firma Wolf (laut Anmelderin im Mai 1988 gedruckt)

## Beschreibung

Die Erfindung geht aus von einem Koagulationsinstrument nach dem Oberbegriff des Patentanspruchs 1. Ein solches Instrument ist in der US-A-4 532 924 offenbart.

Nasenblutungen sind nicht nur auf lokale Ursachen wie zum Beispiel auf Frakturen des Nasenseptums, sondern häufig auch auf pathologische Veränderungen, wie Arteriosklerose oder dergleichen, zurückzuführen und bedürfen somit der ärztlichen Behandlung, zumal die Epistaxis für das Leben des davon betroffenen Patienten ein nicht zu unterschätzendes Risiko darstellt.

Im Gegensatz zu den meisten im vorderen Bereich der Nasenhöhle auftretenden Blutungen sind Blutungen im hinteren Nasenbereich schwierig zu behandeln, da diese im absteigenden Gefäßbereich des nasopharyngealen Venengeflechtes und der zugehörigen Keilbeinartherienzweige einerseits schwierig einzusehen sind und andererseits gerade dort größere Blutungen auftreten.

Um derartige Blutungen zu stillen, ist es nach dem Stand der Technik (Katalogblätter G 20, V. 88; G 21, V.88 der Anmelderin) erforderlich, eine Endoskopoptik und getrennt hiervon ein Koagulations-Sauginstrument an den Ort der Blutung heranzuführen, um die Blutung unter visueller Kontrolle durch Koagulation zu stillen. Wegen der teilweise vorhandenen Notwendigkeit, jedes zur Blutungsstelle führende Blutgefäß koagulieren zu müssen, ist die Ausführung der erforderlichen Koagulation bei gleichzeitiger optischer Kontrolle sehr umständlich und belastet den Patienten wie auch den Operateur in erheblichem Maße, da dieser die beiden Instrumententeile mit seinen beiden Händen halten bzw. betätigen muß, wobei sich diese in keiner festen Beziehung zueinander befinden und daher unabhängig voneinander bewegt werden müssen.

In dem vorgenannten Dokument USA- 4 532 924 ist ein elektrochirurgisches Instrument zur Koagulation von Blutgefäßen beschrieben, das einen kombinierten flexiblen Koagulations-Spül- und Saugkatheter mit einer Koagulationselektrode an seinem distalen Ende und eine Endoskopoptik aufweist. Durch den Schaft dieses Katheters ist eine Spülflüssigkeit ein- und abführbar. In einem weiteren Dokument DE-A-24 26 781 ist ein im Zusammenhang mit einem Endoskop zu. verwendender Katheter beschrieben, der einen flexiblen Schlauch mit einem darin geführten, mit HF-Energie gespeisten Federdraht umfaßt. Der Federdraht kann axial bewegt werden, um das distale, als Sonde ausgebildete Ende des Schlauches ablenken zu können. Durch die Sonde ist ein Kontrastmittel an den Therapieort einleitbar.

Ausgehend von einem Koagulationsinstrument, bestehend aus einer Endoskopoptik und einem Koagulations-Sauginstrument, liegt der Erfindung die Aufgabe zugrunde, ein solches Koagulationsinstrument so auszubilden, daß die Arbeit des Arztes zum Koagulieren, insbesondere von Blutungen in den Nasenhöhlen, wesentlich erleichert wird, und darüberhinaus die Möglichkeit zu schaffen, auch schwierig einzusehende Körperhöhlenbereiche, wie den hinteren Nasenbereich, unter visueller Kontrolle und erforderlichenfalls unter Verwendung zusätzlicher Hilfsinstrumente auf einfache Art und Weise gezielt therapieren zu können.

Die Lösung dieser Aufgabe ist in dem Patentanspruch 1 angegeben.

Durch diese Lösung kann der Arzt das eine Einheit bildende Instrument mit einer Hand erfassen und mit der anderen, freien Hand die Optik und den Koagulations-Spül- und Saugkatheter oder zusätzlich verwendete Hilfsinstrumente bedienen, so daß seine Arbeit wesentlich erleichtert wird und sicherer durchgeführt werden kann. Weiter ist der Koagulations-Spül- und Saugkatheter geeignet, ein flüssiges Spülmittel mit an die Koagulationsstelle zu führen und auch die Spülflüssigkeit, Körpersekrete oder dergleichen abzusaugen.

Ein Koagulationsinstrument nach der Erfindung ist nachstehend anhand der Zeichnung beispielsweise beschrieben. Es zeigen:
- Fig. 1: eine Draufsicht auf das Koagulationsinstrument,
- Fig. 2: das Instrument nach Fig. 1 in Seitenansicht,
- Fig. 3: einen Querschnitt längs der Linie III-III in Fig. 2,
- Fig. 4: einen durch das Instrument hindurchführbaren Koagulations-Spül- und Saugkatheter in Seitenansicht,
- Fig. 5, 6 und 7: drei Querschnitte längs der Linie IV-IV in Fig. 4 mit unterschiedlicher Kanalzahl innerhalb der Wandung des Katheters.

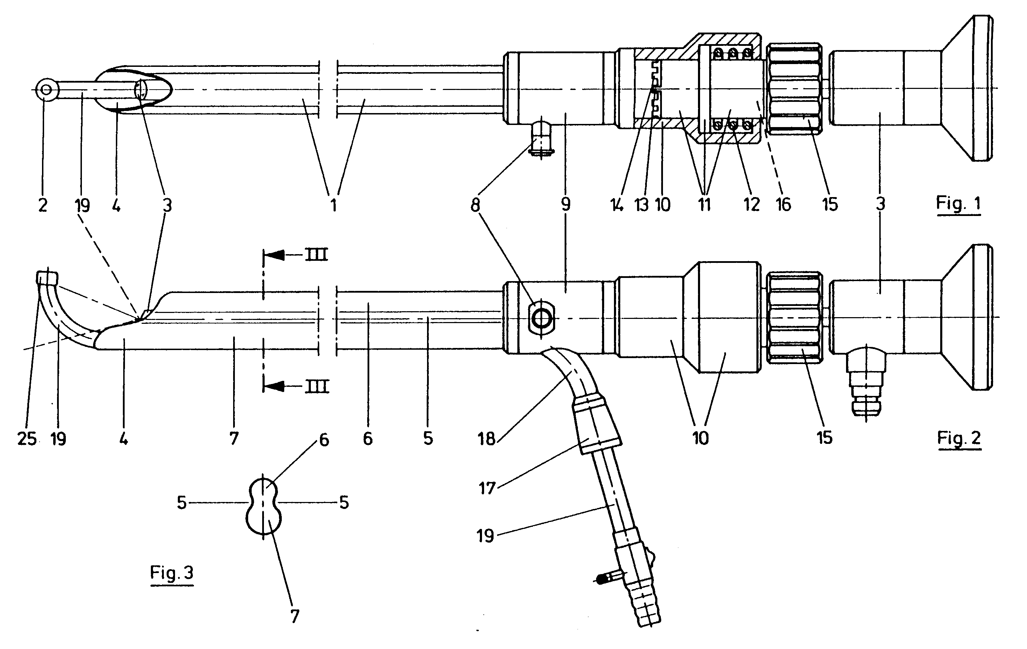

Das Koagulationsinstrument nach Fig. 1 besteht aus einem Schaft 1, dessen distal offenes Ende in seinem unteren Bereich durch eine Schaftwandverlängerung eine in etwa U-förmige Führung 4 für das distale Ende eines weiter unten erläuterten Katheters 19 bildet. Der obere und untere Schaftbereich sind, wie in Fig. 3 dargestellt, durch seitliche, entlang der Schaftlängsachse verlaufende Umfangseinschnürungen 5 in zwei auf der Länge ineinander übergehende Kanäle 6, 7 aufgeteilt, wobei der obere Kanal 6 einen kleineren Durchmesser als der untere Kanal 7 aufweist. Am proximalen, im Durchmesser erweiterten Schaftende 9 ist ein drehbarer Anschlußstutzen 8 zum Zu- und Abführen einer Spülflüssigkeit oder dergleichen angeordnet. An die Schafterweiterung 9 schließt sich ein Kupplungsgehäuse 10 an, in dem eine mittels einer Druckfeder 12 vorgespannte Aufnahme 11 um die Längsachse verdrehbar und um eine bestimmte Länge axial verschiebbar angeordnet ist. Am distalen Ende der Aufnahme 11 befindet sich ein Stift 13 oder dergleichen, der in eine von entsprechenden Radialnuten 14 im Inneren des Schaftgehäuses 1 am distalen Ende des Schaftes 1 eingreift, um eine mittels eines Spannrings 15 lösbar festgelegte Endoskopoptik 3 in einer bestimmten Ausblickposition zu fixieren. Die Anordnung der Ausblickposition kann dadurch erfolgen, daß die mit der Aufnahme 11 gekuppelte Optik 3 um eine bestimmte Länge proximalwärts gegen die Federung 12 gezogen und in dieser Position um die Längsachse in die erforderliche bzw. gewünschte Ausblickrichtung gedreht wird. Im Anschluß daran kann die an der Optik 3 angreifende Zugkraft aufgehoben werden, wodurch die Optik distalwärts unter gleichzeitigem Eingreifen des Stiftes 13 in eine der kranzförmig angeordneten Nuten 14 distalwärts bewegt wird und in dieser Stellung lösbar festgelegt ist.

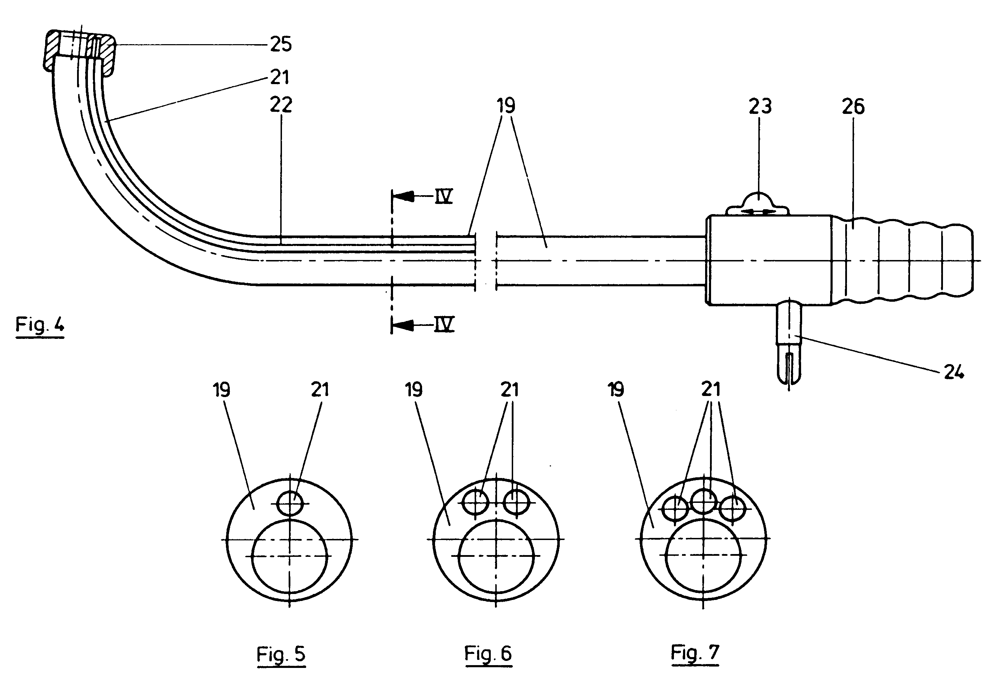

Da der Durchmesser der Endoskopoptik 3 verhältnismäßig gering gewählt werden kann, kann zu deren Führung im Schaft 1 der obere Kanal 6 kleineren Durchmessers Verwendung finden. Dadurch besteht die Möglichkeit, durch den unteren Kanal 7 ein Hilfsinstrument mit verhältnismäßig großem Außendurchmesser hindurchzuführen, vor allem ein Koagulations-Spül- und Saugkatheter nach Fig. 4. Das Einführen des Katheters nach Fig. 4 erfolgt durch einen an der Unterseite in den Kanal 7 mündenden, mittels einer Gummidichtung 17 flüssigkeitsdicht angeordneten Anschlußstutzen 18.

Ein in Verbindung mit dem Schaft 1 zu verwendendes Instrument ist beispielhaft in der Figur 4 dargestellt und zeigt einen Koagulations-Spül- und Saugkatheter 19. Dieser Katheter besteht im wesentlichen aus einem Schaftrohr aus flexiblem Material, welches je nach Verwendungszweck eine höhere oder geringere Flexibilität hat. Um auch schwer zugängliche Stellen in der tiefliegenden Nasenhöhle problemlos behandeln zu können, ist das distale Ende 2 des Katheters zumindest in einer Ebene steuerbar ausgebildet. Die steuerbare Ablenkung erfolgt hierbei mittels eines in einem in der Wand des Schaftrohres befindlichen Kanal 21 geführten Zugdrahtes 22, der an seinem distalen Ende mit einer Elektrode 25 des Schaftrohres und proximal mit dem zur Ablenkung erforderlichen Betätigungselement 23 verbunden ist, wobei das Ablenken des distalen Katheterendes durch Verschieben eines Elementes 23 erfolgt. In einfachster Ausbildung des Katheters 19 kann der zum Ablenken des distalen Katheterendes an dessen als Koagulationselektrode 25 ausgebildeten distalen Endteiles angreifende Zugdraht 22 zur Übertragung des HF-Stromes verwendet werden. Bei Ausbildung des Katheters in einfachster, vorstehend erläuterter Ausbildung weist das Schaftrohr den in Figur 5 dargestellten Querschnitt auf.

Werden bei monopolarer Ausbildung des Katheters 19 für die Zuführung des HF-Stromes und zur Kathetersteuerung gesonderte Drähte verwendet, so kann das Schaftrohr entsprechend dem Querschnitt nach Figur 6 ausgebildet sein. Hierbei ist jedoch auch eine bipolare Ausbildung des Katheters denkbar, wobei dann in jedem der beiden vorhandenen Kanäle 21 ein Leiter zur Zuführung des HF-Stromes angeordnet sein kann. Einer der beiden Leiter muß dann, wie im ersteren Beispiel nach Figur 5 beschrieben ist, gleichzeitig zur Ablenkung des distalen Katheterendes 2 benutzt werden.

Durch die Anordnung von drei getrennten Kanälen 21 nach Figur 7 in der Wandung des Schaftrohres besteht die Möglichkeit, sowohl jedem der beiden HF-Stromzuführungen als auch dem Zugdraht einen Kanal zuzuordnen. Die Zuführung des HF-Stromes erfolgt über einen HF-Anschluß 24. Proximal ist der Katheter 19 mit einem Anschlußstutzen 26 für einen aufschiebbaren Schlauch versehen, mit dem eine Saug- und Spülpumpe zur Zu- und Abführung einer Spülflüssigkeit verwendbar ist.

Anstelle einer Ablenkung des distalen Katheterendes durch den Zugdraht 22 kann der aus einem flexiblen Kunststoff hergestellte Katheter auch so ausgebildet sein, daß in einem der Kanäle 21 ein Gedächtnismetall integriert ist, durch das das distale Ende beispielsweise aus der gestreckten Ruhelage abgelenkt werden kann.

## Patentansprüche

1. Koagulationsinstrument, bestehend aus einer Endoskopoptik (3) und einem Koagulations-Spül-Saugkatheter (19) mit einer Koagulationselektrode (25) an seinem distalen Ende, insbesondere zur Stillung von Blutungen im Bereich der Nasenhöhlen, dadurch gekennzeichnet, daß ein Schaft (1) einen Kanal (6) zur Durchführung der Endoskopoptik (3) und einen Kanal (7) zur Durchführung eines flexiblen Koagulations-Spül- und Saugkatheters (19) mit distalem, durch eine proximale Handhabe (23) in wenigstens einer Ebene ablenkbaren Ende (2) aufweist und daß durch den Schaft (1) und/oder durch den Spül-Saugkatheter (19) eine Spülflüssigkeit zu- und/oder abführbar ist.

2. Instrument nach Anspruch 1, dadurch gekennzeichnet, daß durch den Koagulations-Spül- und Saugkatheter (19) mit distaler Koagulationslelektrode (25) wenigstens ein Kanal (21) zur Durchführung eines auch zur Ablenkung des distalen Katheterendes dienenden HF-Stromleiters (22) zu der distalen Koagulationselektrode (25) verläuft.

3. Instrument nach Anspruch 2, dadurch gekennzeichnet, daß innerhalb der Wandung des Koagulations-Spül- und Saugkatheters (19) zwei Kanäle (21) für zwei HF-Stromleiter verlaufen.

4. Instrument nach Anspruch 3, dadurch gekennzeichnet, daß innerhalb der Wandung des Koagulations-Spül- und Saugkatheters (19) zwei Kanäle (21) für zwei HF-Stromleiter und ein dritter Kanal (21) für einen Draht zum Ablenken des distalen Katheterendes verlaufen.

5. Instrument nach einem der Ansprüche 1 bis 4, dadurch gekennzeichnet, daß der durch den Schaft (1) verlaufende Kanal (7) für den Koagulations-Spül- und Saugkatheter (19) einen größeren Durchmesser hat als der Kanal (6) für die Endoskopoptik (3) und daß beide Kanäle (7, 6) in Längsrichtung miteinander verbunden sind.

6. Instrument nach einem der Ansprüche 1 bis 5, dadurch gekennzeichnet, daß der flexible Koagulations-Spül- und Saugkatheter (19) an seinem distalen Ende mit einem Zugdraht ablenkbar ist.

7. Instrument nach einem der Ansprüche 1 bis 5, dadurch gekennzeichnet, daß in einem der Kanäle (21) oder innerhalb der Schaftwandung des flexiblen Koagulations-Spül- und Saugkatheters (19) im Bereich des distalen Endes ein Gedächtnismetall integriert ist, mit dem das distale Katheterende (2) ablenkbar ist.

8. Instrument nach einem der Ansprüche 1 bis 7, dadurch gekennzeichnet, daß die distalwärts durch eine Feder (12) vorgespannte Endoskopoptik (3) mit seitlichem Ausblick durch axiales Zurückziehen aus ihrer Riegelstellung bringbar und zur Änderung der Ausblickrichtung verdrehbar ist.

9. Instrument nach Anspruch 8, dadurch gekennzeichnet, daß die federvorgespannte Endoskopoptik (3) nach erfolgter Einstellung der Blickrichtung und Freigabe der an ihr angreifenden, proximalwärts gerichteten Zugkraft durch Eingriff eines Stiftes (13) oder dergleichen in radiale Nuten (14) lösbar festlegbar ist.

10. Instrument nach einem der Ansprüche 1 bis 9, dadurch gekennzeichnet, daß der Koagulations-Spül- und Saugkatheter (19) ein im Winkel zur Katheterlängsachse abgewinkeltes distales Ende (2) aufweist, das zum Durchführen durch den Kanal (7) des Schaftes (1) in eine gestreckte Lage bringbar ist.

## Claims

1. Coagulation instrument comprising an endoscope optical system (3) and a coagulation rinsing and suction catheter (19) having a coagulation electrode (25) on its distal end, particularly for stopping bleeding in the region of nasal cavities, characterised in that a shaft (1) has a channel (6) for insertion of the endoscope optical system (3) and a channel (7) for insertion of a flexible coagulation rinsing and suction catheter (19) having a distal end (2) which can be deflected by means of a proximal handle (23) in at least one plane, and in that a rinsing liquid can be supplied and/or discharged through the shaft (1) and/or through the rinsing and suction catheter (19).

2. Instrument according to claim 1, characterised in that at least one channel (21) for insertion of an HF current conductor (22) also serving to deflect the distal end of the catheter extends to the distal coagulation electrode (25) through the coagulation rinsing and suction catheter (19) having distal coagulation electrode (25).

3. Instrument according to claim 2, characterised in that two channels (21) for two HF current conductors extend within the wall of the coagulation rinsing and suction catheter (19).

4. Instrument according to claim 3, characterised in that two channels (21) for two HF current conductors and a third channel (21) for a wire for deflecting the distal end of the catheter extend within the wall of the coagulation rinsing and suction catheter (19).

5. Instrument according to one of claims 1 to 4, characterised in that the channel (7) for the coagulation rinsing and suction catheter (19) extending through the shaft (1) has a greater diameter than the channel (6) for the endoscope optical system (3), and in that both channels (7, 6) are connected to one another in the longitudinal direction.

6. Instrument according to one of claims 1 to 5, characterised in that the flexible coagulation rinsing and suction catheter (19) can be deflected at its distal end using an extension wire.

7. Instrument according to one of claims 1 to 5, characterised in that a memory metal, by means of which the distal end (2) of the catheter can be deflected, is integrated into one of the channels (21) or within the shaft wall of the flexible coagulation rinsing and suction catheter (19) in the region of the distal end.

8. Instrument according to one of claims 1 to 7, characterised in that the endoscope optical system (3) with lateral viewfinder and pretensioned in the distal direction by means of a spring (12) can be taken from its locking position by means of axial withdrawal and can be twisted to change the viewfinder direction.

9. Instrument according to claim 8, characterised in that the spring-pretensioned endoscope optical system (3) can be releasably fixed, after completing adjustment of the viewing direction and releasing the tensile force directed proximally and acting on it, by engaging a pin (13) or the like in radial grooves (14).

10. Instrument according to one of claims 1 to 9, characterised in that the coagulation rinsing and suction catheter (19) has a distal end (2) offset at a 90° angle to the longitudinal axis of the catheter and which can be brought into an extended position for insertion through channel (7) of shaft (1).

## Revendications

1. Instrument de coagulation, constitué par un système optique endoscopique (3) et un cathéter de lavage-aspiration - coagulation (19) comportant une électrode de coagulation (25) à son extrémité distale, notamment pour arrêter des hémorragies dans la région des cavités nasales, caractérisé en ce qu'une tige (1) comporte un canal (6) pour le passage du système optique endoscopique (3) et un canal (7) pour le passage d'un cathéter flexible de lavage-aspiration - coagulation (19) comportant une extrémité distale (2) qui peut être déviée dans au moins un plan, à l'aide d'un élément de manipulation proximal (23), et en ce qu'un liquide de lavage peut être amené et/ou évacué à travers la tige (1) et/ou à travers le cathéter de lavage et d'aspiration (19).

2. Instrument selon la revendication 1, caractérisé en ce qu'à travers le cathéter de lavage - aspiration - coagulation (19) possédant une électrode distale de coagulation (25), s'étend au moins un canal (21) pour le passage d'un conducteur de courant HF (22), également utilisé pour la déviation de l'extrémité distale du cathéter, jusqu'à l'électrode distale de coagulation (25).

3. Instrument selon la revendication 2, caractérisé en ce que deux canaux (21) pour deux conducteurs de courant HF s'étendent à l'intérieur de la paroi du cathéter de lavage - aspiration - coagulation (19).

4. Instrument selon la revendication 3, caractérisé en ce que deux canaux (21) pour deux conducteurs de courant HF et un troisième canal (21) pour un fil métallique servant à dévier l'extrémité distale du cathéter, s'étendent à l'intérieur de la paroi du cathéter lavage - aspiration - coagulation (19).

5. Instrument selon l'une des revendications 1 à 4, caractérisé en ce que le canal (7), qui s'étend à travers la tige (1), pour le cathéter de lavage - aspiration - coagulation (19) possède un diamètre supérieur à celui du canal (6) pour le système optique endoscopique (3) et en ce que les deux canaux (7, 6) sont reliés entre eux dans la direction longitudinale.

6. Instrument selon l'une des revendications 1 à 5, caractérisé en ce que le cathéter flexible lavage - aspiration - coagulation (19) peut être dévié à son extrémité distale, par un fil de traction.

7. Instrument selon l'une des revendications 1 à 5, caractérisé en ce que, dans l'un des canaux (21) ou à l'intérieur de la paroi de la tige du cathéter flexible de lavage - aspiration - coagulation (19), est intégré, dans la région de l'extrémité distale, un métal à mémoire de forme, à l'aide duquel l'extrémité distale (2) du cathéter peut être déviée.

8. Instrument selon l'une des revendications 1 à 7, caractérisé en ce que le système optique endoscopique (3), qui est précontraint, vers le côté distal, par un ressort (12) et qui a une visée latérale, peut être dégagé de sa position verrouillée, par retrait axial et peut être tourné pour la modification de la direction de visée.

9. Instrument selon la revendication 8, caractérisé en ce qu'une fois le réglage de la direction de visée effectué et après suppression de la force de traction qui lui est appliquée et qui est dirigée vers le côté proximal, le système optique endoscopique (3), précontraint par un ressort, peut être immobilisé de façon libérable par engagement d'un téton (13) ou analogue dans des rainures radiales (14).

10. Instrument selon l'une des revendications 1 à 9, caractérisé en ce que le cathéter de lavage - aspiration -coagulation (19) présente une extrémité distale (2), qui est coudée suivant un certain angle par rapport à l'axe longitudinal du cathéter et peut être amenée dans une position étirée, pour passer à travers le canal (7) de la tige (1).
